# EUROPÄISCHE PATENTANMELDUNG

(11) **EP 0 700 682 A2**
(43) Veröffentlichungstag der Anmeldung: **13.03.1996**
(21) Anmeldenummer: 95112569.9
(22) Anmeldetag: 10.08.1995
(51) Int. Cl.: A61K 38/18

(54) **Arzneimittel, enthaltend Prothrombin zur Antagonisierung von Blut-Anticoagulanzien**

(30) Priorität: 26.08.1994 DE 4430204
(71) Anmelder: BEHRINGWERKE Aktiengesellschaft, D-35001 Marburg (DE)
(72) Erfinder: Römisch, Jürgen, Dr., D-35041 Marburg (DE); Diehl, Karl-Heinz, Dr., D-35418 Buseck-Oppenrod (DE); Stöhr, Hans-Arnold, D-35083 Wetter (DE)

(57) **Zusammenfassung**

Offenbart wird ein Arzneimittel, welches als Gegenmittel für Blut-Antikoagulanz dient und Prothrombin enthält.

## Beschreibung

Gegenstand der Erfindung ist ein Gegenmittel (Antidot) für Blut-Antikoagulanzien.

Die Blutgerinnung ist ein komplexer Prozeß, an dem Plasmaproteine und Zellen beteiligt sind. Die Bildung eines Wundverschlusses wird durch die rasche Adhäsion von Blutplättchen an die geschädigten Oberflächen und durch Proteasen und Kontaktfaktoren eingeleitet, die nacheinander in einer kaskadenartigen Reaktionsabfolge aktiviert werden. Am Ende dieser Vorgänge steht die Erzeugung von Thrombin, das unter anderem die Aktivierung von Fibrinogen zu Fibrin katalysiert.

Proteine wie die Faktoren VIII und V tragen als Akzelleratoren zur Effizienz dieses Systems bei. Neben dem Protein C-System regulieren Inhibitoren der beteiligten Proteasen im wesentlichen die Gerinnungsprozesse dahingehend, daß ein Wundverschluß zustandekommt, jedoch überschießende Reaktionen vermieden werden. Wird dieses fein abgestimmte Gleichgewicht gestört, kann dies Fibrinablagerungen bzw. die Bildung von Thrombosen zur Folge haben. Bekanntermaßen sind resultierende Gefäßverschlüsse die Ursache für viele pathophysiologische Prozesse, wie insbesondere den Herzinfarkt. Diese Problematik bekommt ein besonderes Gewicht bei solchen Patienten, die einer erhöhten Thrombosegefahr ausgesetzt sind, was häufig eine postoperative Komplikation darstellt.

Zur Verhinderung dieser pathophysiologischen Prozesse werden verschiedene Wege beschritten. So werden zur Verhinderung der Thrombusbildung bzw. zur Unterstützung deren Auflösung (Thrombolyse) in der Klinik häufig Antikoagulanzien angewendet, die in unterschiedlicher Art und Weise in das Gerinnungsgeschehen eingreifen. Neben der Reduktion bzw. Verhinderung der Thrombozytenfunktionen (Aggregation, Adhäsion, Aktivierung, Synthese und Ausschüttung von Aktivierungsprodukten) werden Hemmstoffe von beteiligten Gerinnungsproteasen, insbesondere des Thromboplastin/Faktor VIIa-Komplexes, des Faktors Xa und insbesondere des Thrombins (Faktor IIa) untersucht und angewendet.

Ein besonders spezifischer und potenter Inhibitor des Thrombins ist das Hirudin, das ursprünglich aus den Speicheldrüsen des Blutegels Hirudo medicinalis isoliert wurde. Mittlerweile sind verschiedene Varianten, Analoga, Fragmente und Mutanten davon bekannt und werden auf ihre Tauglichkeit untersucht. In seiner natürlich vorkommenden Form ist das Hirudin sulfatiert, jedoch wurde festgestellt, daß die interessante biologische Aktivität auch bei der nicht sulfatierten Form erhalten bleibt. Daher kann das Hirudin bzw. die Hirudin-Derivate in bequemer Weise mit den Mitteln der Gentechnologie hergestellt werden.

Zu den Blut-Antikoagulanzien zählt auch das Heparin, dessen Fragmente und Varianten sowie synthetische Hemmstoffe, die die katalytische Aktivität des Thrombins und anderer Gerinnungsproteasen, z.B. Faktor Xa, inhibieren. Auch diese Komponenten können bei der Prophylaxe und/oder Therapie von thrombotischen bzw. präthrombotischen Komplikationen eingesetzt werden. Die Suche nach spezifischeren und potenteren Antikoagulanzien bzw. Antithrombotika reflektiert die unbefriedigende Situation, daß die klinisch etablierten Substanzen wie Heparine oder Vitamin K-Antagonisten entweder nicht effektiv genug sind oder, daß sie unerwünschte und zum Teil gravierende Nebenwirkungen aufweisen. Hirudin ist der stärkste derzeit bekannte Thrombininhibitor, der auch nicht in die anderen Enzyme der Blutgerinnungskaskade eingreift. Bisher wurden keine wesentlichen Nebeneinflüsse auf die Herzschlagrate, die Atmung, den Blutdruck, die Anzahl der Thrombozyten, den Fibrinogen- oder Hämoglobingehalt beobachtet. Daher handelt es sich bei Hirudin um ein bevorzugtes Blut-Antikoagulationsmittel.

Die Anwendung von Antikoagulanzien bzw. Antithrombotika am Patienten bringt naturgemäß das Risiko einer erhöhten Blutungsneigung mit sich, die in extremen Situationen, z.B. bei unsachgemäßer Anwendung bzw. Dosierung zu unkontrollierten Blutungen führen kann. Um in einer derartigen Situation der unerwünschten Blutungsneigung gegensteuern zu können, ist es bei einigen Substanzen, deren Beschaffenheit (Molekulargewicht, Struktur) es erlaubt, möglich, diese schnell durch Dialyse aus dem Blutplasma des Patienten zu entfernen. Wenn dies aber aufgrund der apparativen Ausstattung oder aus sonstigen Gründen nicht möglich ist, muß ein Gegenmittel, das auch als Antidot bezeichnet wird, zur Verfügung gestellt werden.

Als Gegenmittel sind bereits einige Substanzen bzw. Präparate vorgeschlagen worden. So zeigen die Thrombine und deren blockierte Varianten einen gewissen Hirudin-neutralisierenden Effekt, jedoch ist einerseits die Anwendung von aktivem Thrombin bei Patienten, die mit Blut-Antikoagulanzien behandelt werden, in der Regel kontra-indiziert und andererseits ist die Präparation von inaktivem Thrombin durch Anwendung niedermolekularer, kovalente Bindungen eingehende Inhibitoren aufgrund der Toxizität dieser Substanzen problematisch. Außerdem fehlen auch Erfahrungen, ob diese Komplexe eine möglicherweise antigene Wirkung besitzen. Diese Komplexe sind auch aufgrund ihrer Natur nicht geeignet, niedermolekulare (synthetische) Antithrombine bzw. Antithrombotika zu neutralisieren. Das Meizothrombin, ein Intermediärprodukt, das bei der Aktivierung vom Prothrombin zu Thrombin kurzfristig entsteht, besitzt zwar eine gewisse Potenz zur Antagonisierung, jedoch kann es sich in vivo schnell zu Thrombin umwandeln und ist als solches in der Regel kontraindiziert.

Die Untersuchung des Faktor VII als Hirudin-Antidot in entsprechenden Modellen erbrachte keine befriedigenden Ergebnisse. In der europäischen Patentanmeldung EP A 89.810733.9 wird die Verwendung von Faktor VIII oder Fragmenten des Faktors VIII als Gegenmittel für Blut-Antikoagulanzien beschrieben. Bekannt ist auch, daß solche Substanzen verwendet werden können, die die Konzentration an Faktor VIII im Blut erhöhen, wie beispielsweise das Desmopressin (Mannucci, P.M.; Progress in Haemostasis and Thrombosis, 8 (1986), S. 19-45).

Da aber auch diese Gegenmittel nicht für alle Anwendungsbereiche ausreichen, besteht nach wie vor die Notwendigkeit, effektivere und an Nebenwirkungen ärmere Gegenmittel für Blut-Antikoagulanzien, Antithrombotika bzw. Plättchenantagonisten bereitzustellen.

Unter antikoagulatorischen bzw. antithrombotischen Substanzen werden im Rahmen der vorliegenden Anmeldung Glykosaminoglykane und sulfatierte Polysaccharide, darunter Heparine, im besonderen unfraktioniertes und niedermolekulares (LMW-) Pentosanpolysulfat, Heparansulfat, Dermatansulfat oder. Condroitinsulfat sowie Hirudine, deren Fragmente, z. B. Hirugen (J. Biol. Chem. 265 (1990), 13484-13487; EP-A-0 333 356), Analoga oder Mutanten, z. B. Hirulog® (WO 92/13952), gekoppelte Hirudine, z. B. PEG-Hirudin oder Hirutonine natürliche oder synthetische Inhibitoren des Thromboplastin/Faktor VIIaKomplexes, des Faktors Xa oder des Thrombins, Komponenten des Protein C-Systems, nämlich aktiviertes Protein C, Protein S, Thrombomodulin, Fibrinaggregations- oder Vernetzungshemmstoffe, Substanzen aus der Vitamin K-Antagonistengruppe, Substanzen, die die Plättchenaktivierung und/oder -aggregation bzw. Plättchenadhäsion beeinflussen, wie insbesondere Fibrinogenrezeptor-Antagonisten, oder die endogene Fibrinolysekapazität zu steigern vermögen, wie insbesondere PAI-1 Inhibitoren oder Synthese- und Sekretionsstimulatoren von Plasminogenaktivator-Inhibitoren und somit indirekt antithrombotisch wirken, verstanden.

Da die genannten gekoppelten Hirudine, z. B. PEG-Hirudin, aus dem Organismus nur schwer entfernbar sind, ist die Bereitstellung eines geeigneten Antidots für gekoppelte Hirudine besonders wichtig.

Überraschenderweise wurde gefunden, daß Prothrombin (Faktor II) hohe Wirksamkeit als Gegenmittel für Blut-Antikoagulanzien besitzt.

Gegenstand der vorliegenden Erfindung ist deshalb ein Arzneimittel, enthaltend Prothrombin zur Antagonisierung von Blut-Antikoagulanzien, wobei diese Blut-Antikoagulanzien ausgewählt sein können aus der Gruppe umfassend Glykosaminoglykane und sulfatierte Polysaccharide sowie Hirudine, deren Fragmente, Analoga oder Mutanten, natürliche und synthetische Inhibitoren des Thromboplastins/Faktor VIIa-Komplexes, des Faktors Xa oder des Thrombins, Komponenten des Protein C-Systems, nämlich aktiviertes Protein C, Protein S, Thrombomodulin, Fibrinaggregations- oder Vernetzungshemmstoffe, Substanzen aus der Vitamin K-Antagonistengruppe, Substanzen, die die Plättchenaktivierung und/oder -aggregation bzw. Plättchenadhäsion beeinflussen, wie insbesondere Fibrinogenrezeptor-Antagonisten, oder die endogene Fibrinolysekapazität zu steigern vermögen, wie insbesondere PAI-1 Inhibitoren oder Synthese- und Sekretionsstimulatoren von Plasminogenaktivator-Inhibitoren.

Unter Prothrombin werden im Rahmen vorliegender Erfindung auch aktive Fragmente oder Varianten verstanden, die konventionell oder insbesondere gentechnologisch hergestellt werden können.

In dem erfindungsgemäßen Arzneimittel wird Prothrombin in einer Konzentration von 1 bis 2000 Internationalen Einheiten (IU) pro Kilogramm Körpergewicht des zu behandelnden Patienten eingesetzt, wobei Konzentrationen von 10 bis 1000 IU/kg besonders bevorzugt sind.

Die Erfindung betrifft auch die Verwendung von Prothrombin als Gegenmittel für Blut-Antikoagulanzien.

Weiterhin betrifft die Erfindung ein Verfahren zur Herstellung eines Arzneimittels, welches Prothrombin enthält zur Antagonisierung von Blut-Antikoagulanzien.

Die vorliegende Erfindung wird durch das nachfolgende Beispiel weiter erläutert.

### Beispiel

Standard-Humanplasma (SHP), das mit 20 µg/ml Hirudin versetzt wurde, wurde in der aPTT auf antagonisierende Wirkung durch Prothrombin (F II) untersucht. Die in der Tabelle demonstrierten Gerinnungszeiten belegen die Wirksamkeit des Prothrombins.

**Tabelle**

| | **aPTT (sec)** |
|---|---|
| Kontrolle | >2000,0 |
| 2 IU Faktor II | 258,7 |
| 2 IU Faktor V | > 2000,0 |
| 2 IU Faktor VII | > 2000,0 |
| 2 IU Faktor VIII | >2000,0 |
| 2 IU Faktor IX | >2000,0 |
| 2 IU Faktor X | > 2000,0 |

## Patentansprüche

1. Arzneimittel, enthaltend Prothrombin zur Antagonisierung von Blut-Antikoagulanzien.

2. Arzneimittel nach Anspruch 1, wobei Prothrombin die Bedeutung von konventionell oder gentechnisch hergestellten aktiven Fragmenten oder Varianten von Prothrombin hat.

3. Arzneimittel nach Anspruch 1, worin Prothrombin in einer Konzentration von 1 bis 2000 Internationalen Einheiten (IU) pro Kilogramm Körpergewicht des zu behandelnden Patienten enthalten ist.

4. Arzneimittel nach Anspruch 3, worin die Prothrombinkonzentration 10 bis 1000 IU/kg Körpergewicht des zu behandelnden Patienten beträgt.

5. Verfahren zur Herstellung eines Arzneimittels gemäß Anspruch 1 zur Antagonisierung von Blut-Antikoagulanzien.
